# EUROPEAN PATENT APPLICATION

(11) **EP 3 943 596 A1**
(43) Date of publication of application: **26.01.2022**
(21) Application number: 20773734.7
(22) Date of filing: 23.03.2020
(51) Int. Cl.: C12N 5/071

(54) **METHOD FOR CULTURING HEPATIC EPITHELIOID TISSUE HAVING A STRUCTURE OF CONNECTIONS BETWEEN HEPATOCYTES AND CHOLANGIOCYTES**

(30) Priority: 20.03.2019 JP 2019052282
(71) Applicant: Sapporo Medical University, Sapporo-shi, Hokkaido 060-8556 (JP)
(72) Inventor: TANIMIZU, Naoki, Sapporo-shi, Hokkaido 060-8556 (JP); MITAKA, Toshihiro, Sapporo-shi, Hokkaido 060-8556 (JP)
(74) Representative: Regimbeau
(86) International application number: PCT/JP2020/014421
(87) International publication number: WO 2020/189799

(57) **Abstract**

There is provided a novel method for culturing a hepatic epithelioid tissue. The method uses a method for forming a hepatic epithelioid tissue having a structure of connections between hepatocytes and bile duct epithelial cells, comprising a step of culturing bile duct epithelial cells on a collagen gel, and a step of inoculating and culturing hepatocytes on the cultured bile duct epithelial cells.

## Description

### CROSS-REFERENCE TO RELATED APPLICATIONS

This application is based upon and claims the benefit of priority from Japanese Patent Application No. 2019-52282, filed on March 20, 2019; the entire contents of which are incorporated herein by reference.

### TECHNICAL FIELD

The present invention relates to a method for forming a hepatic epithelioid tissue, and more specifically a method for forming a hepatic epithelioid tissue having a structure of connections between hepatocytes and bile duct epithelial cells, the method comprising a step of culturing bile duct epithelial cells on a collagen gel, and a step of inoculating and culturing hepatocytes on the cultured bile duct epithelial cells.

### BACKGROUND ART

Many therapeutic drugs for diseases are metabolized by hepatocytes in the liver to exhibit physiological activity or toxicity. Therefore, in preclinical experiments for development of novel drugs, it is desirable to conduct activity/toxicity studies using hepatocytes that exhibit high metabolic activity equivalent to that in vivo. It is also necessary to construct assay systems reflecting the pharmacokinetics in liver tissues.

It is described that liver tissue spheroids whose functions are similar to those in vivo have been produced by using a mixture of hepatocytes, endothelial cells, and stem cells (e.g., Patent Document 1). However, in the spheroid culture method, although certain effects are exhibited in order to enhance the functions of hepatocytes, the tissue structure of the liver, especially the hepatic epithelial tissue structure, had not been reconstructed, and thus the pharmacokinetics at the tissue/organ level had not been adequately reproduced.

### RELATED ART DOCUMENT

### Patent Literature

Patent Literature 1 : JP 2016-105700 A

### SUMMARY OF THE INVENTION

The present inventors have found that, by using a certain method for forming a hepatic epithelial tissue, it is possible to reconstruct ex vivo a hepatic epithelioid tissue structure having a structure of connections between hepatocytes and bile duct epithelial cells, which is similar to that in vivo. The present invention is based on these findings.

Therefore, the present invention provides a method for forming a hepatic epithelioid tissue in which it is possible to reconstruct ex vivo a hepatic epithelioid tissue structure having a structure of connections between hepatocytes and bile duct epithelial cells, which is similar to that in vivo.

According to the present invention, the following inventions are provided:
(1) A method for forming a hepatic epithelioid tissue having a structure of connections between hepatocytes and bile duct epithelial cells, comprising the following steps of:
   a step of culturing bile duct epithelial cells on a collagen gel; and
   a step of inoculating and culturing hepatocytes on the cultured bile duct epithelial cells.
(2) The method for forming a hepatic epithelioid tissue according to (1), wherein the collagen gel comprises 2 to 6 mg/mL of collagen.
(3) The method for forming a hepatic epithelioid tissue according to (1) or (2), wherein the hepatocytes are inoculated and cultured on the bile duct epithelial cells having a density of 20 to 40% of the cultured region as a result of culture on the collagen gel.
(4) The method for forming a hepatic epithelioid tissue according to any one of (1) to (3), further comprising a step of culturing by overlaying a gel comprising an EHS-gel after the culture step of inoculating and culturing hepatocytes.
(5) The method for forming a hepatic epithelioid tissue according to (4), wherein the gel comprises the EHS-gel at a concentration of 5% (v/v) or more.
(6) The method for forming a hepatic epithelioid tissue according to any one of (1) to (5), wherein the hepatocytes are small hepatocytes and/or mature hepatocytes.
(7) The method for forming a hepatic epithelioid tissue according to any one of (1) to (5), wherein the hepatocytes are murine small hepatocytes and/or murine mature hepatocytes.
(8) The method for forming a hepatic epithelioid tissue according to any one of (1) to (5), wherein the hepatocytes are human liver progenitor cells.
(9) A hepatic epithelioid tissue formed by the method for forming a hepatic epithelioid tissue according to any one of (1) to (8).
(10) A liver tissue comprising the hepatic epithelioid tissue according to (9).
(11) A liver tissue reconstructed ex vivo having a structure of connections between hepatocytes and bile duct epithelial cells.
(12) The liver tissue according to (10) or (11), wherein one or more markers selected from the group consisting of the following (a) to (d) are expressed:
   (a) one or more hepatocyte metabolic function markers selected from the group consisting of Cps, Tdo2, Cyp, and Ugt1a1;
   (b) one or more bile duct epithelial cell transporter markers selected from the group consisting of Ae2 and Cftr;
   (c) one or more bile duct epithelial cell cytoskeleton markers selected from the group consisting of Ck7 and Ck19; and
   (d) Cldn4 being a molecule that forms a tight junction of a bile duct.
(13) The liver tissue according to any one of (10) to (12), wherein one or more hepatocyte metabolic function markers selected from the group consisting of Cps, Tdo2, Cyp, and Ugt1a1 are expressed.
(14) The liver tissue according to any one of (10) to (13), wherein one or more bile duct epithelial cell transporter markers selected from the group consisting of Ae2 and Cftr are expressed.
(15) The liver tissue according to any one of (10) to (14), wherein one or more bile duct epithelial cell cytoskeleton markers selected from the group consisting of Ck7 and Ck19 are expressed.
(16) The liver tissue according to any one of (10) to (15), wherein Cldn4 being a molecule that forms a tight junction is expressed.
(17) The liver tissue according to any one of (10) to (16), which is used as a disease model.
(18) The liver tissue according to (17), wherein the disease model is a fatty liver model.

It is advantageous that, by using the method for forming a hepatic epithelioid tissue of the present invention, it is possible to reconstruct ex vivo a hepatic epithelioid tissue structure having a structure of connections between hepatocytes and bile duct epithelial cells, which is similar to that in vivo.

### BRIEF DESCRIPTION OF THE DRAWINGS

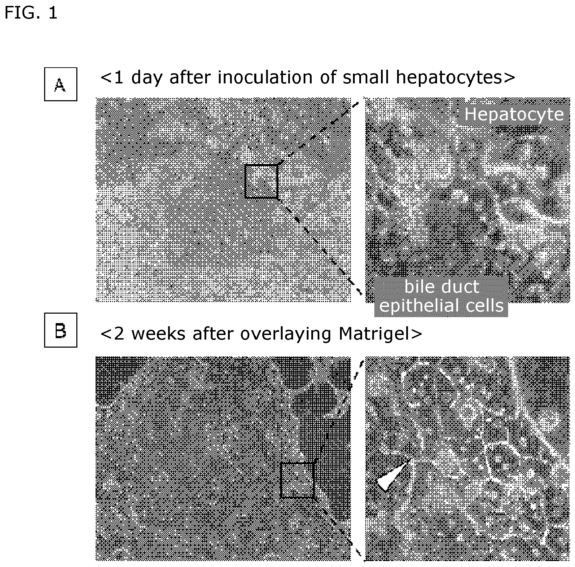

FIG. 1A represents an image 1 day after inoculation of small hepatocytes. FIG. 1B represents an image 2 weeks after overlaying a collagen gel containing Matrigel (registered trademark). The right figures represent enlarged views of respective parts enclosed in a box, and the arrowhead shows a structure of connection between hepatocytes and bile duct epithelial cells.

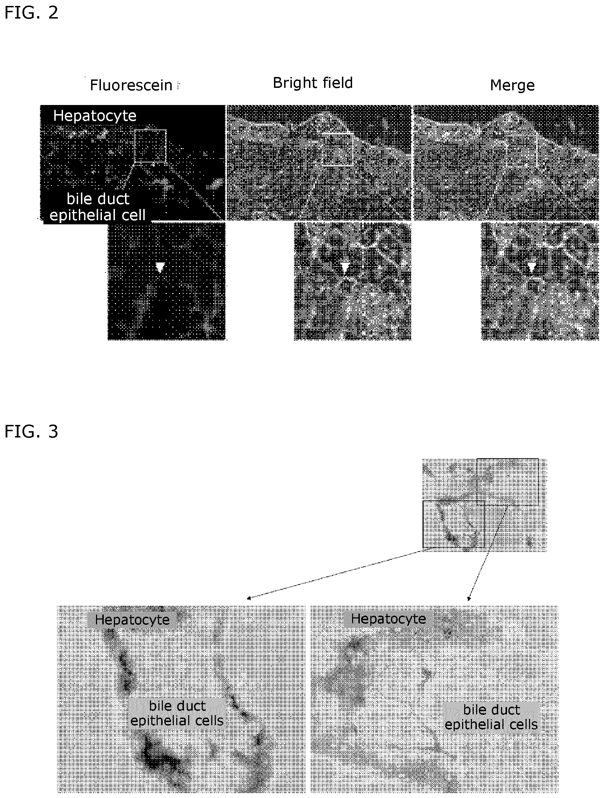

FIG. 2 shows that fluorescein diacetate taken up by hepatocytes was decomposed in the hepatocytes, leading to release of fluorescein being a fluorescence substance, to flow into a bile duct structure formed by bile duct epithelial cells. The left figure represents an image observed with a fluorescence microscope (indicated as "Fluorescein" in the figure), the middle figure represents an image observed with a phase-contrast microscope (indicated as "Bright field" in the figure), and the right figure represents a figure obtained by merging an image observed with a fluorescence microscope with an image observed with a usual microscope (indicated as "Merge" in the figure) (the arrowheads in the figures show connections between hepatocytes and bile duct epithelial cells).
FIG. 3 represents an upper right figure before enlargement, and images obtained by enlargement of respective parts enclosed in a box in each site of the upper right figure, showing that bilirubin flows into a bile duct structure formed by bile duct epithelial cells.

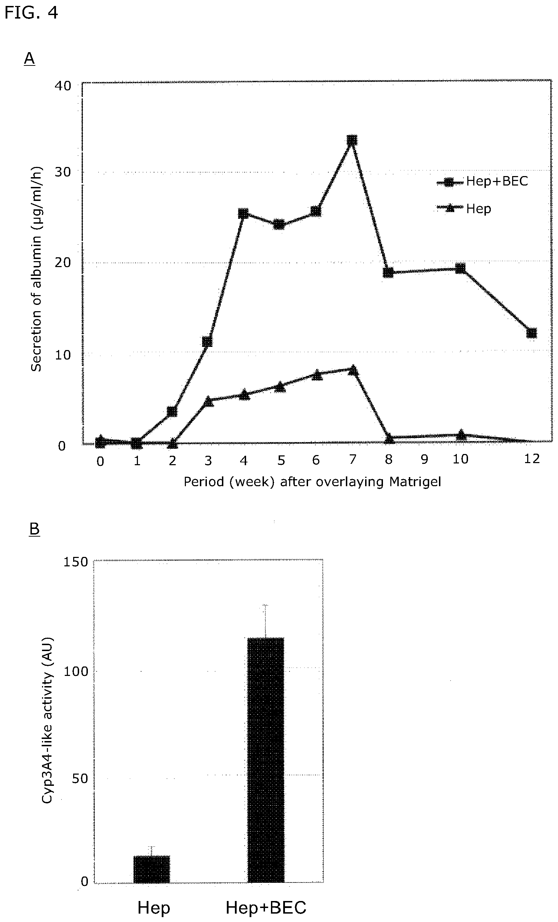

FIG. 4A represents the secretion amount (µg/ml/h) of albumin over time after overlaying a type I collagen gel containing 20% (v/v) Matrigel (after overlaying Matrigel). FIG. 4B represents the Cyp3A4-like activity (AU) in small hepatocytes (Hep) (monoculture of small hepatocytes) or a hepatic epithelioid tissue 2 weeks after overlaying Matrigel (Hep + BEC) (coculture of small hepatocytes and bile duct epithelial cells).

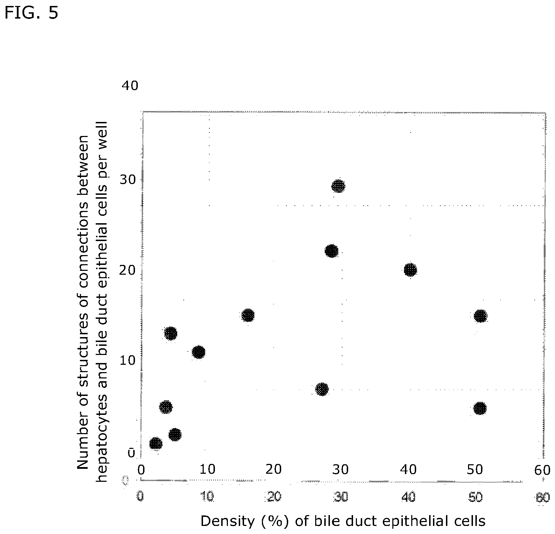

FIG. 5 represents the relationship between the size of a bile duct epithelial cell colony and formation of a hepatic epithelioid tissue. The vertical axis represents the number of structures of connections between hepatocytes and bile duct epithelial cells formed per well, and the horizontal axis represents the density (%) in the cultured region of bile duct epithelial cells.

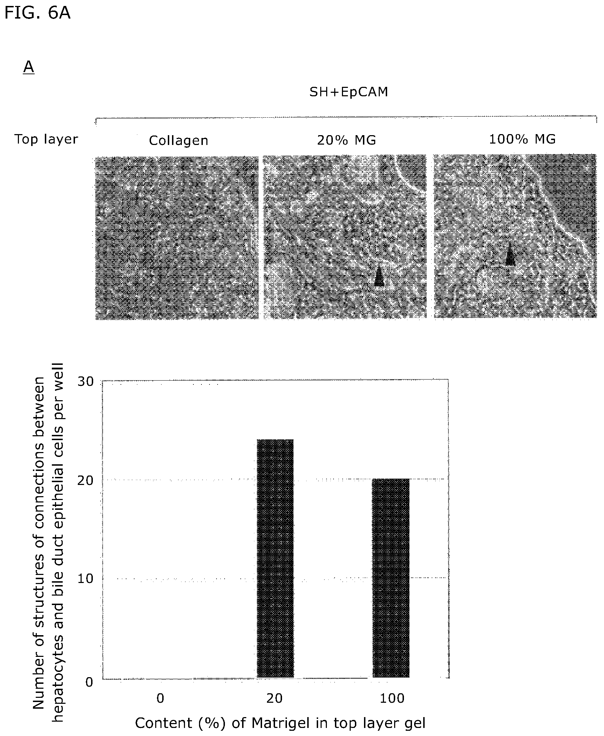

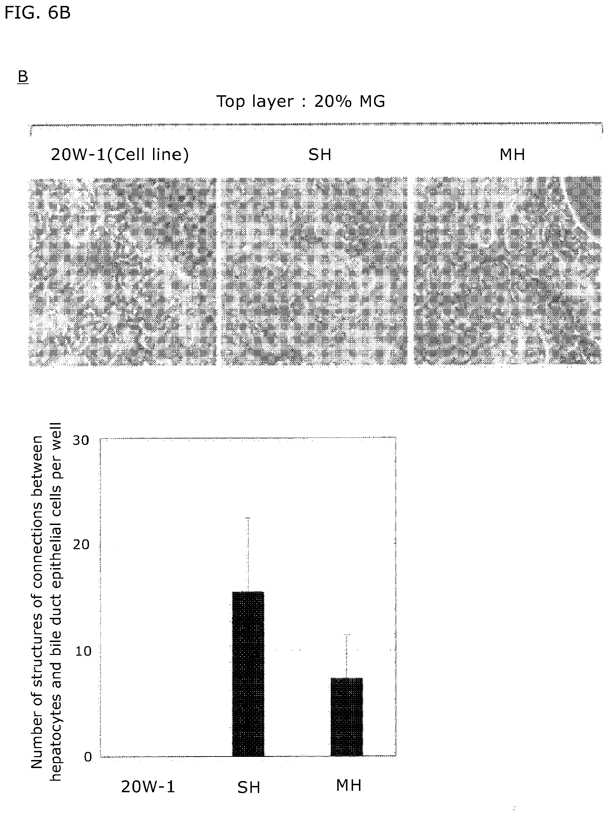

FIG. 6A represents images of observation of hepatic epithelioid tissues formed using a collagen gel only (Collagen) (left), a collagen gel containing 20% (v/v) Matrigel (20% MG) (middle), and 100% (v/v) Matrigel (100% MG) (right) as a top layer gel to be overlaid (Top layer). The arrowheads show a structure of connections between hepatocytes and bile duct epithelial cells. The graph of FIG. 6A represents the number of structures of connections between hepatocytes and bile duct epithelial cells relative to the content (%) of Matrigel in the top layer gel. FIG. 6B represents images of observation of hepatic epithelioid tissues formed by using 20W-1 (liver progenitor cells) (left), SH (small hepatocytes) (middle), and MH (mature hepatocytes) (right). As a top layer gel to be overlaid (Top layer), a collagen gel containing 20% (v/v) Matrigel (20% MG) was used. The graph of FIG. 6B represents the number of structures of connections between hepatocytes and bile duct epithelial cells formed per well (vertical axis) in 20W-1, SH, and MH (horizontal axis).

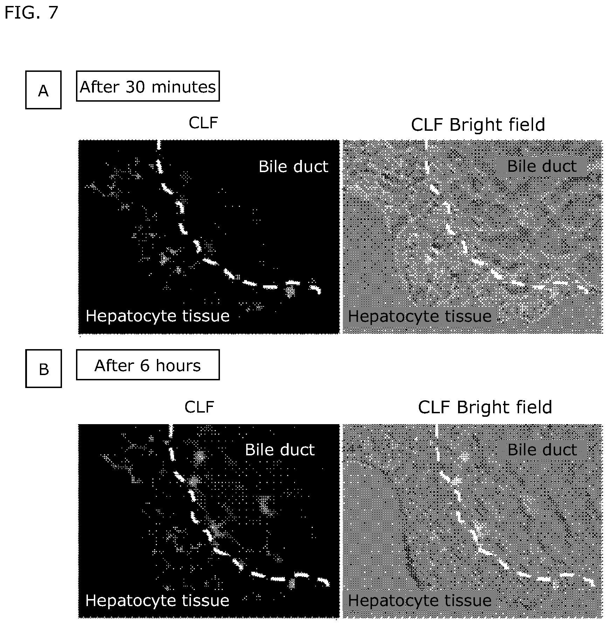

The left figure of FIG. 7A is an image showing that cholyl lysine fluorescein (CLF) being a bile acid analog was taken up by hepatocytes and excreted into the bile canaliculus (the right figure of FIG. 7A represents an image obtained by merging the fluorescence image of the left figure with a bright-field image). The left figure of FIG. 7B is an image showing that cholyl lysine fluorescein excreted into the bile canaliculus was transported to the bile duct (the right figure of FIG. 7B represents an image obtained by merging the fluorescence image of the left figure with a bright-field image).

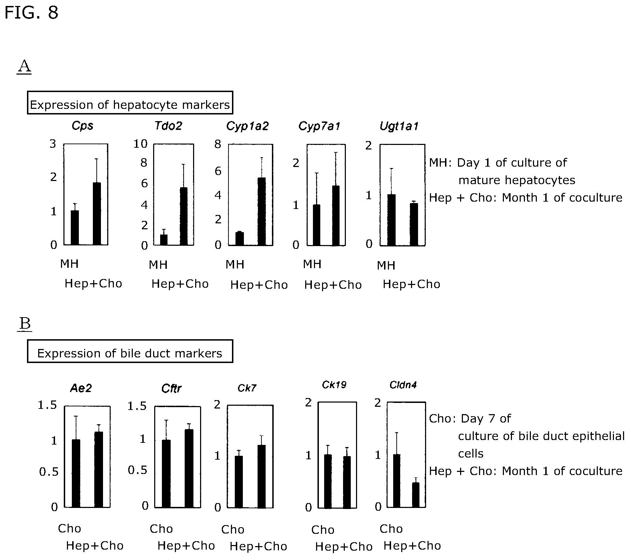

FIG. 8A is a graph comparing the expression level of hepatocyte markers on day 1 of culture of mature hepatocytes (MH) with that in month 1 of coculture (Hep + Cho). FIG. 8B is a graph comparing the expression level of bile duct markers on day 7 of culture of bile duct epithelial cells (Cho) with that in month 1 of coculture (Hep + Cho).

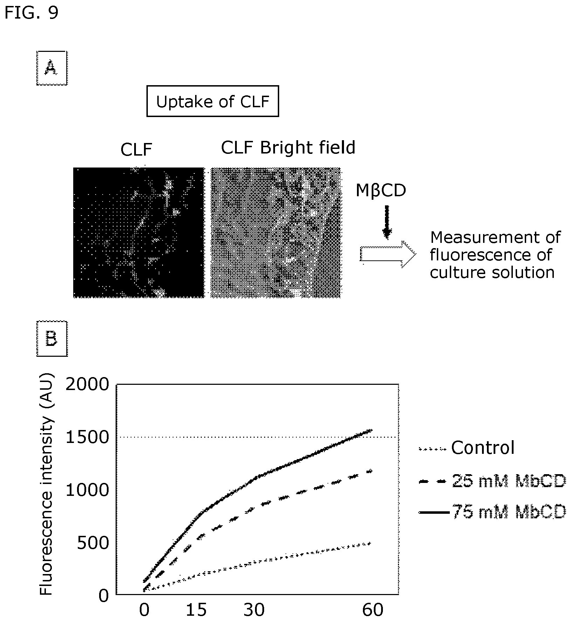

The left figure of FIG. 9A is an image showing a state in which a bile acid analog (CLF) was taken up by a hepatic epithelioid tissue (the right figure of FIG. 9A represents an image obtained by merging the fluorescence image of the left figure with a bright-field image). FIG. 9B is a graph representing the fluorescence intensity of CLF detected after being leaked into the medium by no addition of methyl-beta-cyclodextrin (MβCD) (control) or addition of 25 mM of MβCD or 75 mM of MβCD.

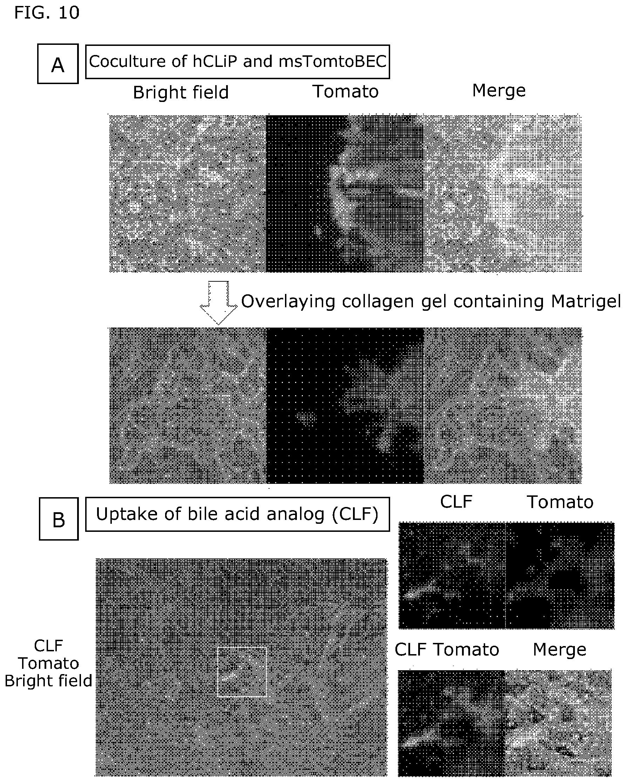

FIG. 10A represents images showing that the contact surface between murine BEC (the range indicated in red) and human CLip (hCLiP) during the early stage of coculture has been formed (before and after overlaying a collagen gel containing Matrigel). The left figure of FIG. 10A represents an image observed with a phase-contrast microscope (indicated as "Bright field" in the figure), the middle figure of FIG. 10A represents an image observed with a fluorescence microscope (indicated as "Tomato" in the figure), and the right figure of FIG. 10A represents a figure obtained by merging an image observed with a fluorescence microscope with an image observed with a usual microscope (indicated as "Merge" in the figure). FIG. 10B shows that CLF has flowed into the bile duct (the range indicated in green) in the vicinity of the contact surface between murine BEC (the range indicated in red) and human CLiP (see the left figure of FIG. 10B), and that CLF taken up by human CLiP has been transported to a murine bile duct tissue (see the right figure of FIG. 10B). The upper right figures of FIG. 10B are images observed with a fluorescence microscope by CLF and Tomato, respectively. The lower right figure of FIG. 10B (left figure) is an image obtained by merging an image of CLF with an image of Tomato, and the lower right figure of FIG. 10B (right figure) is a figure obtained by merging the lower right figure of FIG. 10B (left figure) with an image observed with a phase-contrast microscope (indicated as "Merge" in the figure). CLF represents a bile acid analog, and Tomato being a fluorescent protein represents bile duct epithelial cells expressing it.

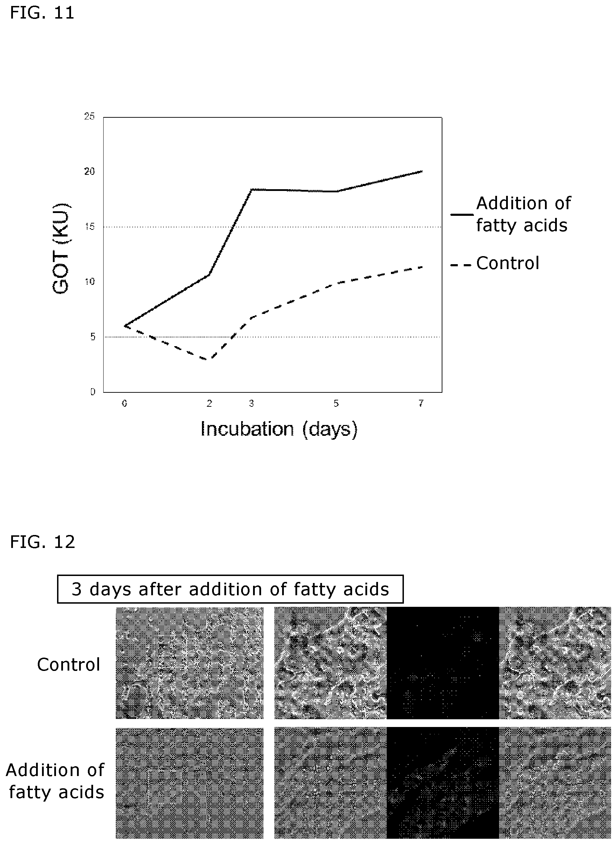

FIG. 11 shows that, when fatty acids are added to a hepatic epithelial tissue, the activity of glutamate oxaloacetate transaminase (GOT) being an injury marker of hepatocytes detected in the medium increases over time depending on the culture time (incubation). The dotted line represents a control without addition of fatty acids (oleic acid and palmitic acid), and the solid line represents one with addition of fatty acids.

FIG. 12 represents a fluorescence microscope image after staining with Nile Red with or without addition of fatty acids.

### DETAILED DESCRIPTION OF THE INVENTION

### [Method for forming a hepatic epithelioid tissue]

The method for forming a hepatic epithelioid tissue of the present invention is a method for forming a hepatic epithelioid tissue having a structure of connections between hepatocytes and bile duct epithelial cells, comprising the following steps of:
a step of culturing bile duct epithelial cells on a collagen gel; and
a step of inoculating and culturing hepatocytes on the cultured bile duct epithelial cells.

A hepatic epithelioid tissue formed by the formation method of the present invention is not particularly limited as long as it is a tissue having at least a structure of connections between hepatocytes and bile duct epithelial cells.

In the method for forming a hepatic epithelioid tissue of the present invention, examples of collagen in a collagen gel used for culture of bile duct epithelial cells include type I collagen, type III collagen, type IV collagen, and type I collagen is preferable. As the type I collagen, products manufactured by Nitta Gelatin Inc., FUJIFILM Corporation, Nippi, Incorporated, and the like may be used, and it is preferable to use collagen manufactured by Corning Incorporated. In the method for forming a hepatic epithelioid tissue of the present invention, the collagen gel used for culture of bile duct epithelial cells can be prepared, for example, by adding the collagen as a solution to wells of a culture plate cooled in advance, followed by allowing to stand for a certain period to be gelatinized.

In the method for forming a hepatic epithelioid tissue of the present invention, the collagen contained in the collagen gel used for culture of bile duct epithelial cells is not limited as long as it is possible to reconstruct ex vivo a hepatic epithelioid tissue structure having a structure of connections between hepatocytes and bile duct epithelial cells, which is similar to that in vivo, and it is contained in an amount of preferably 2 to 6 mg/mL, more preferably 3 to 5 mg/mL, and further preferably 4 mg/mL.

Bile duct epithelial cells (also referred to as BEC) are cells that are positive for cytokeratin-19 (CK19) and epithelial cell adhesion molecule (EpCAM), which are BEC markers, and that express transcription factors such as hepatocyte nuclear factor 1 beta (HNF1b) and grainyhead-like 2 (GRHL2).

As conditions for culturing bile duct epithelial cells on a collagen gel, usual culture conditions for culturing cells have no particular problem, and, for example, it is preferable to culture at 36 to 38°C, and preferably 37°C. As the culture period, as mentioned below, it is preferable to culture bile duct epithelial cells until the density becomes 20 to 40% of the cultured region, and it is preferable to culture for about 3 to 7 days.

According to a preferred embodiment t of the method for forming a hepatic epithelioid tissue of the present invention, there is provided a method for forming a hepatic epithelioid tissue, wherein the hepatocytes are inoculated and cultured on the bile duct epithelial cells having a density of 20 to 40% (preferably 25 to 35%) of the cultured region (e.g., the area of the well of the culture plate) as a result of culture on the collagen gel. A method for measuring the density of the cultured region of bile duct epithelial cells is not particularly limited, and, for example, the density can be measured with image analysis software (manufactured by Olympus Corporation) using photographs taken with a microscope. It is advantageous that, by inoculating and culturing hepatocytes on the bile duct epithelial cells having a density of 20 to 40% of the cultured region, it is possible to increase the number of structures of connections between hepatocytes and bile duct epithelial cells in the formed liver tissue, to further reconstruct ex vivo a hepatic epithelioid tissue structure having a structure of connections between hepatocytes and bile duct epithelial cells, which is similar to that in vivo.

Hepatocytes used in the method for forming a hepatic epithelioid tissue of the present invention are not particularly limited as long as they are cells derived from the liver, and they are preferably small hepatocytes and/or mature hepatocytes, more preferably small hepatocytes or mature hepatocytes, and further preferably small hepatocytes. Here, the small hepatocytes and the mature hepatocytes are preferably murine small hepatocytes and murine mature hepatocytes, respectively.

According to another embodiment of the hepatocytes used in the method for forming a hepatic epithelioid tissue of the present invention, human liver progenitor cells are preferable, and human reprogramming hepatocytes (human chemically induced hepatocyte progenotors: hCLiP) are more preferable.

Here, the small hepatocytes does not mean merely small cells derived from the liver, but they mean a special type of small cells derived from the liver that can be isolated from the liver, that show almost the same phenotype as of mature hepatocytes for markers such as albumin, transferrin, CK8, and CK18, and that have ultrastructural characteristics as hepatocytes but have high proliferative capacity, which have been reported in Mitaka et al, Hepatology, 16, 440-447 (1992), Mitaka et al, Hepatology, 29, 111-135 (1999), WO 01/092481 A, WO 02/088332 A, and WO 2006/001473 A, and the like.

The small hepatocytes have lower specific gravity than that of mature hepatocytes (hepatic parenchymal cells), and when a suspension containing a liver-derived cell population obtained by collagenase treatment, etc., of liver tissues is centrifuged at low gravitational acceleration, the mature hepatocytes are mainly transferred to the precipitate, while the small hepatocytes have the nature of being mainly transferred to the supernatant. In order to efficiently separate small hepatocytes and mature hepatocytes, the centrifugation is preferably performed at 50 × g for 1 minute or less.

The small hepatocytes express CD44, while mature hepatocytes and hepatic nonparenchymal cells do not express it. Due to such characteristics, it is understood that the small hepatocytes are cells different from mature hepatocytes and hepatic nonparenchymal cells.

According to a preferred embodiment of the method for forming a hepatic epithelioid tissue of the present invention, there is provided a method for forming a hepatic epithelioid tissue having a structure of connections between hepatocytes and bile duct epithelial cells, further comprising a step of culturing by overlaying a gel comprising an EHS-gel after the culture step of inoculating and culturing hepatocytes. By adding such a step, it is possible to further reconstruct ex vivo a hepatic epithelioid tissue structure having a structure of connections between hepatocytes and bile duct epithelial cells, which is similar to that in vivo.

The period from inoculation of hepatocytes to further overlaying a gel containing an EHS-gel is not particularly limited, and it is preferable to overlay after culture for several days, and it is more preferable to overlay after 2 to 3 days.

The culture period during which culture is performed by overlaying a gel containing an EHS-gel is not particularly limited as long as it is a period during which a hepatic epithelioid tissue is formed, and, for example, it is preferable to culture at 37°C for at least 1 week, preferably at least 1 to 2 weeks, and more preferably at least 2 weeks.

The EHS-gel is a gel containing basement membrane components purified from murine sarcoma (Engelbreth-Holm-Swarm: EHS), and can be prepared as mentioned in, for example, Kleinman et al. "Basement membrane complexes with biological activity" Biochemistry. 1986 Jan 28;25(2):312-8. As a commercially available EHS-gel, for example, EHS-gel Basement Membrane Matrix (manufactured by FUJIFILM Wako Pure Chemical Corporation), ECM Gel from Engelbreth-Holm-Swarm murine sarcoma (manufactured by Sigma), or Matrigel (manufactured by Corning Incorporated) can be used. The EHS-gel of the present invention is preferably a gel containing a laminin protein, and the gel containing the EHS-gel contains a laminin protein at a concentration of preferably 40 to 80%, and more preferably 50 to 65%. As the EHS-gel containing a laminin protein, Matrigel (manufactured by Corning Incorporated) can be suitably used. Here, the laminin is a protein in which α-chains, β-chains, and γ-chains are associated at a ratio of 1:1:1, and it is known that five types of α-chains, two types of β-chains, and two types of γ-chains exist in humans.

The EHS-gel contained in the gel to be overlaid is not limited as long as it is possible to reconstruct ex vivo a hepatic epithelioid tissue structure having a structure of connections between hepatocytes and bile duct epithelial cells, which is similar to that in vivo, and it is preferably contained at a concentration of 5% (v/v) or more, and more preferably 10% (v/v) or more. The "gel containing an EHS-gel" to be overlaid may contain only an EHS-gel, but when the "gel containing an EHS-gel" contains a gel other than an EHS-gel, a collagen gel is suitably contained, and a type I collagen gel (preferably a collagen gel manufactured by KOKEN CO., LTD) is more suitably contained.

According to another embodiment of the present invention, there is provided a hepatic epithelioid tissue formed by the method for forming a hepatic epithelioid tissue of the present invention. The hepatic epithelioid tissue formed by the method for forming a hepatic epithelioid tissue of the present invention is one that a hepatic epithelioid tissue structure having a structure of connections between hepatocytes and bile duct epithelial cells, which is similar to that in vivo, was reconstructed ex vivo.

According to a preferred embodiment of the present invention, there is provided a method for forming a hepatic epithelioid tissue having a structure of connections between hepatocytes and bile duct epithelial cells, comprising:
a step of culturing bile duct epithelial cells on a collagen gel comprising 2 to 6 mg/mL of collagen;
a step of inoculating and culturing hepatocytes (preferably small hepatocytes or mature hepatocytes) on the bile duct epithelial cells having a density of 20 to 40% of the cultured region as a result of culture on the collagen gel; and
a step of culturing by overlaying a gel comprising an EHS-gel.

According to another embodiment of the present invention, there is provided a hepatic epithelioid tissue formed by the method for forming a hepatic epithelioid tissue of the present invention.

### [Liver tissue]

According to one embodiment of the present invention, a liver tissue containing the hepatic epithelioid tissue formed by the above-mentioned method for forming a hepatic epithelioid tissue of the present invention is formed. Since the hepatic epithelioid tissue formed by the method for forming a hepatic epithelioid tissue of the present invention has a structure of connections between hepatocytes and bile duct epithelial cells, according to another embodiment of the present invention, there is provided a liver tissue reconstructed ex vivo having a structure of connections between hepatocytes and bile duct epithelial cells.

The above-mentioned liver tissue is not particularly limited, and is preferably a liver tissue in which one or more markers selected from the group consisting of the following (a) to (d) are expressed:
(a) one or more hepatocyte metabolic function markers selected from the group consisting of Cps, Tdo2, Cyp, and Ugt1a1;
(b) one or more bile duct epithelial cell transporter markers selected from the group consisting of Ae2 and Cftr;
(c) one or more bile duct epithelial cell cytoskeleton markers selected from the group consisting of Ck7 and Ck19; and
(d) Cldn4 being a molecule that forms a tight junction of a bile duct.

Here, the Cps is carbamoyl phosphate synthetase; the Tdo2 is tryptophan 2,3-dioxygenase; the Cyp is cytochrome P450, preferably Cypla2 or Cyp7a1; the Ugt1a1 is UDP-glucuronosyl-transferase-1 family polypeptide A11a1; the Ae2 is anion exchanger 2; the Cftr is cystic fibrosis transmembrane conductance regulator; the Ck7 is cytokeratin 7; the Ck19 is cytokeratin 19; and the Cldn4 is claudin 4.

According to a preferred embodiment of the present invention, there is provided a liver tissue in which one or more hepatocyte metabolic function markers selected from the group consisting of Cps, Tdo2, Cyp, and Ugt1a1 are expressed.

According to a preferred embodiment of the present invention, there is provided a liver tissue in which one or more bile duct epithelial cell transporter markers selected from the group consisting of Ae2 and Cftr are expressed.

According to a preferred embodiment of the present invention, there is provided a liver tissue in which one or more bile duct epithelial cell metabolic function markers selected from the group consisting of Ck7 and Ck19 are expressed.

According to a preferred embodiment of the present invention, there is provided a liver tissue in which Cldn4 being a molecule that forms a tight junction is expressed.

According to a more preferred embodiment of the present invention, there is provided a liver tissue in which all markers of Cps, Tdo2, Cyp, Ugt1a1, Ae2, Cftr, Ck7, Ck19, and Cldn4 are expressed.

The above-mentioned liver tissue of the present invention can be used as a disease model. By using the liver tissue of the present invention, any model can be produced using a known method as long as the disease is derived from a liver tissue, and, for example, a fatty liver model, a model of acute hepatic disorder due to drugs, etc., a hepatic fibrogenesis model, etc., are exemplified, of which preferably a fatty liver model can be utilized. The fatty liver model can be produced by adding oleic acid and/or palmitic acid to the liver tissue of the present invention.

In the construction of a fatty liver model that has already been reported, spheroid culture has often been performed (e.g., see Patent Document 1). Therefore, in order to check the morphology of hepatocytes to confirm that fat droplets are accumulated in the cytoplasm, it is necessary to prepare a section or to perform observation with a confocal microscope, etc. In the fatty liver model using the liver tissue of the present invention, the morphology of hepatocytes can be easily observed, and thus it is possible to show simply and clearly that fat droplets are accumulated in the cytoplasm.

According to a more preferred embodiment of the present invention, the disease model of the liver tissue of the present invention can be used for screening for therapeutic drugs for the disease, preferably fatty liver, acute hepatic disorder due to drugs, or hepatic fibrogenesis, and thus suitably a method for screening for therapeutic drugs is provided.

### EXAMPLES

The present invention will be more specifically described by way of Examples, but the technical scope of the present invention is not limited thereto.

### 1. Separation of cells from the liver

Collagenase perfusion was performed for the liver of male mice aged 8 to 10 weeks purchased from Sankyo Labo Service Corporation, INC., and thus obtained cell suspension was centrifuged at 50 x g for 1 minute. The cells obtained by centrifuging the supernatant at 115 x g for 3 minutes were suspended in Hanks' Balanced Salt Solution (HBSS) (manufactured by Sigma Aldrich Co. LLC), and an equal amount of Percoll (manufactured by GE Healthcare Japan Corporation) was added thereto, followed by centrifugation at 180 x g for 15 minutes to remove dead cells, and thus obtained cells were used as small hepatocytes.

The undigested tissue after collagenase perfusion was cut into small pieces, and the small pieces were stirred in a collagenase and hyaluronidase solution to be digested. After an antibody (manufactured by BioLegend, Inc.) against EpCAM being a surface marker of bile duct epithelial cells was reacted, EpCAM(+) cells were separated by using MACS (manufactured by Miltenyi Biotec Inc.). These were used as bile duct epithelial cells in the following procedures.

### 2. Culture

After 4 mg/ml of a type I collagen solution was prepared, it was added to wells of a pre-cooled culture plate (manufactured by Greiner Bio-One Co., Ltd.), followed by allowing to stand at 37°C for 30 to 60 minutes to be gelatinized. Bile duct epithelial cells suspended in a DMEM/F12 medium (manufactured by Sigma Aldrich Co. LLC) containing 10% serum (fetal bovine serum), 1 x insulin-transferrin-selenium (ITS), 0.1 µM dexamethasone, 5 ng/ml epidermal growth factor (EGF), and 5 ng/ml hepatocyte growth factor (HGF) were inoculated in a 24-well plate at 5 × 10⁴ cells/well. Culture was performed at 37°C for about 4 to 5 days, and when bile duct epithelial cells had a density of about 30% of the cultured region, small hepatocytes were inoculated at 5 × 10⁴ cells/well. The density in the cultured region of the bile duct epithelial cells in the cultured region was measured with image analysis software (manufactured by Olympus Corporation) by using photographs taken under a microscope. After culture at 37°C for 2 days, 10 ng/ml recombinant murine oncostatin M (mOSM) (manufactured by R&D Systems Inc.) was added, and further after 1 day, 1.6 mg/ml of a type I collagen gel containing 20% (v/v) Matrigel (registered trademark) (manufactured by Corning Incorporated) (EHS-gel) was overlaid. After allowing to stand at 37°C for 3 to 4 hours to be gelatinized, a DMEM/F12 medium (manufactured by Sigma Aldrich Co. LLC) containing serum, 1 x ITS, dexamethasone, and 1% DMSO was added. Culture was continued while performing medium replacement once a week.

### Test Example 1: Confirmation of a structure of connections between hepatocytes and bile duct epithelial cells

Images 1 day after inoculation of small hepatocytes and 2 weeks after overlaying a type I collagen gel containing 20% (v/v) Matrigel (after overlaying Matrigel) are shown in FIG. 1. In the image 2 weeks after overlaying Matrigel, a structure of connections between hepatocytes and bile duct epithelial cells was clearly observed (see FIG. 1B).

### Test Example 2: Confirmation of a structure of connections between hepatocytes and bile duct epithelial cells

Fluorescein diacetate (FDA) (manufactured by Tokyo Chemical Industry Co., Ltd.) was taken up by the above-mentioned hepatic epithelioid tissue 2 weeks after overlaying Matrigel, and observation was performed with a phase-contrast microscope and a fluorescence microscope. In the above-mentioned hepatic epithelioid tissue 2 weeks after overlaying Matrigel, a structure of connections between hepatocytes and bile duct epithelial cells (arrowhead) has been formed, showing that the fluorescein has flowed into the bile duct (see FIG. 2). FDA is taken by hepatocytes and hydrolyzed to become fluorescein that emits a fluorescence, and then is excreted into the bile canaliculus.

### Test Example 3: Uptake of bilirubin by the hepatic epithelioid tissue reconstructed ex vivo

After the above-mentioned hepatic epithelioid tissue 2 weeks after overlaying Matrigel was cultured for 3 to 5 days in a medium containing 10 µg/ml of bilirubin (manufactured by FUJIFILM Wako Pure Chemical Corporation) and fixed in neutral formalin, Fouchest's reagent ((20% trichloroacetic acid (TCA) (manufactured by FUJIFILM Wako Pure Chemical Corporation)/1% ferric chloride (manufactured by FUJIFILM Wako Pure Chemical Corporation)) was added to develop a color, followed by observation with a microscope. As shown in FIG. 3, it was found that bilirubin taken up by hepatocytes flows into a bile duct structure formed by bile duct epithelial cells.

### Test Example 4: Investigation of coculture on hepatocyte functions associated with formation of a structure of connections between hepatocytes and bile duct epithelial cells

The amount of albumin secreted from small hepatocytes obtained by culturing only small hepatocytes at 37°C for 2 weeks under the same conditions as for coculture (Hep) (monoculture of small hepatocytes) and the above-mentioned hepatic epithelioid tissue 2 weeks after overlaying Matrigel (Hep + BEC) (coculture of small hepatocytes and bile duct epithelial cells) was quantitatively determined by the sandwich ELISA method using an anti-albumin antibody (manufactured by Bethyl Laboratories, Inc.). As a result, it was found that, for about 1 month from 2 weeks after overlaying Matrigel, a sufficient albumin secretion amount was observed in Hep + BEC, and liver differentiation functions were maintained (see FIG. 4A).

For the above-mentioned Hep and the above-mentioned Hep + BEC, the Cyp3A4 activity being an index of cytochrome P450 activity was measured with P450-Glo CYP3A4 Assay System (manufactured by Promega Corporation). As a result, it was found that Hep + BEC had higher Cyp3A4 activity compared with Hep, and that higher liver functions are induced in Hep + BEC compared with Hep (see FIG. 4B).

### Test Example 5: Investigation of the relationship between the size of a bile duct epithelial cell colony and formation of a hepatic epithelioid tissue

In the above-mentioned "2. Culture", small hepatocytes were inoculated when bile duct epithelial cells had a density of about 30% of the cultured region, but in order to investigate the optimal timing of inoculation of small hepatocytes, densities other than the above-mentioned density were also investigated. Specifically, the timing of inoculation of small hepatocytes was changed using the density of the cultured region of bile duct epithelial cells as an index, and the number of structures of connections between hepatocytes and bile duct epithelial cells per well of the hepatic epithelioid tissue 2 weeks after overlaying Matrigel was measured with image analysis software (manufactured by Olympus Corporation) by using photographs taken under a microscope. According to the measurement results, it was found that, after hepatocytes are inoculated when bile duct epithelial cells had a density of about 30% of the cultured region, the formation number of structures of connections between hepatocytes and bile duct epithelial cells is increased, and it is possible to better reconstruct ex vivo a hepatic epithelioid tissue structure having a structure of connections between hepatocytes and more satisfactorily, which is similar to that in vivo (see FIG. 5).

### Test Example 6: Investigation of the relationship between the composition of a gel to be overlaid and the type of hepatocytes used for coculture and formation of a hepatic epithelioid tissue

The relationship between the content (%) of an EHS-gel (Matrigel) in a gel to be overlaid and the number of structures of connections between hepatocytes and bile duct epithelial cells formed in a hepatic epithelial tissue was investigated. Specifically, as the proportion of Matrigel used in the above-mentioned "2. Culture", three concentrations of 0% (v/v), 20% (v/v), and 100% (v/v) were investigated, and as a result, it was found that, when Matrigel is contained at a concentration of 20% (v/v) or 100% (v/v) in a gel to be overlaid, the number of structures of connections between hepatocytes and bile duct epithelial cells shows high values, and that even when only an EHS-gel (Matrigel) is used, it is possible to reconstruct ex vivo a hepatic epithelioid tissue structure having a structure of connections between hepatocytes and bile duct epithelial cells, which is similar to that in vivo (see FIG. 6A).

Next, the type of hepatocytes used for coculture with bile duct epithelial cells was investigated. Specifically, when mature hepatocytes (MH) or a liver progenitor cell line (20W-1) established from small hepatocytes of mice aged 20 weeks were/was used instead of small hepatocytes (SH) used in the above-mentioned "2. Culture", effects on the formation number of structures of connections between hepatocytes and bile duct epithelial cells in the hepatic epithelioid tissue formed 2 weeks after overlaying Matrigel were investigated. When the mature hepatocytes were used, the formation number of structures of connections between hepatocytes and bile duct epithelial cells was good although it was slightly lower than that when small hepatocytes were used. On the other hand, when the liver progenitor cell line was used, it was found that no structure of connections between hepatocytes and bile duct epithelial cells was formed at all (see FIG. 6B).

### Test Example 7: Investigation of the collagen concentration in a gel used for the lower layer

Effects of the collagen concentration in a gel used for culturing bile duct epithelial cells first on the ability to form a structure of connections between hepatocytes and bile duct epithelial cells formed in a hepatic epithelioid tissue were investigated. Specifically, after preparing 2, 4, or 6 mg/ml as the collagen concentration in a gel used for culturing bile duct epithelial cells first in the above-mentioned "2. Culture", effects on the ability to form a structure of connections between hepatocytes and bile duct epithelial cells in the hepatic epithelioid tissue formed 2 weeks after overlaying Matrigel were investigated. As a result, all concentrations of 2, 4, and 6 mg/ml showed good ability to form a structure of connections between hepatocytes and bile duct epithelial cells, and especially 4 mg/ml showed the best ability to form a structure of connections between hepatocytes and bile duct epithelial cells (data not shown). It is known that bile duct epithelial cells have low proliferation efficiency and are dedifferentiated even when cultured on a usual cell culture dish.

### Test Example 8: Investigation of transportation of metabolites from hepatocytes to the bile duct

In the above-mentioned "2. Culture", at 1 month after overlaying 1.6 mg/ml of a type I collagen gel containing an EHS-gel, CLF being a bile acid analog was added to the medium to perform culture, and as a result, it was found that CLF was taken up by hepatocytes and excreted into the bile canaliculus (after 30 minutes), and then transported to the bile duct (after 6 hours). FIG. 7A is an image showing that CLF was taken up by hepatocytes and excreted into the bile canaliculus, and FIG. 7B is an image showing that CLF excreted into the bile canaliculus has been transported to the bile duct.

From these findings, it was found that, in addition to FDA and bilirubin, bile acid is also taken up by hepatocytes and then transported to the bile duct.

### Test Example 9: Investigation of expression of hepatocyte and bile duct epithelial cell markers by organoid-constituting cells

For the mature hepatocytes (MH) same as used in Test Example 6 on day 1 of culture (indicated as "MH" in FIG. 8A) and the liver tissue containing the hepatic epithelioid tissue mentioned above in month 1 of coculture of bile duct epithelial cells and small hepatocytes in the above-mentioned "2. Culture" (indicated as "Hep + Cho" in FIG. 8A), the gene expression level of Cps, Tdo2, Cypla2, Cyp7a1, and Ugt1a1, which are hepatocyte metabolic function markers, was compared. For the bile duct epithelial cells (Cho) used in the above-mentioned "2. Culture" on day 7 of culture (indicated as "Cho" in FIG. 8B) and the liver tissue containing the hepatic epithelioid tissue mentioned above in month 1 of coculture of bile duct epithelial cells and small hepatocytes in the above-mentioned "2. Culture" (indicated as "Hep + Cho" in FIG. 8B), the expression level of Ae2 and Cftr being bile duct epithelial cell transporter markers, Ck7 and Ck19 being bile duct epithelial cell cytoskeleton markers, and Cldn4 being a molecule that forms a tight junction was compared. The results are shown in FIG. 8A and FIG. 8B.

The results of FIG. 8A revealed that, in this culture system, Cps, Tdo2, Cyp, and Ugt1a1 being hepatocyte metabolic function markers have been equally expressed without significant difference between the expression level in the hepatic epithelioid tissue and the expression level in the mature hepatocytes.

The results of FIG. 8B revealed that, in this culture system, the bile duct epithelial cell transporters (Ae2, Cftr), the cytoskeletons (Ck7, Ck19), and the molecule that forms a tight junction (Cldn4) have been equally expressed without significant difference between the expression level of the hepatic epithelioid tissue and the expression level of the bile duct epithelial cells.

### Test Example 10: Investigation of recovery of metabolites due to inhibition of functions of cell-cell adhesion

Methyl beta cyclodextrin (MβCD) has been reported to be a substance having an action to pull out cholesterol from the cell membrane (Christoroforides E et al. Org. Chem 2018). Claudin, which constitutes a tight junction, is localized in the membrane domain that is rich in cholesterol, and thus pulled out from the cell membrane by addition of MβCD (Shigetomi K et al. J Cell Biol.2018).

In the above-mentioned "2. Culture", when a bile acid analog (CLF) is added to the cells 1 month after addition of an EHS-gel, CLF is taken up by intracellular (green fluorescence region) to accumulate in the lumen in the organoid (see FIG. 9A), and when MβCD was added thereto, fluorescence was detected in the medium (see FIG. 9B). This is because the barrier functions of the epithelial cells in the organoid are inhibited, resulting in release of CLF in the medium, and it was found that this release depends on the concentration of MβCD to be added (see FIG. 9B).

### Test Example 11: Investigation of coculture of human reprogramming hepatocytes and murine BEC

In order to produce an organoid reflecting the metabolic functions of humans, coculture of human reprogramming hepatocytes and murine bile duct epithelial cells was performed. Specifically, murine bile duct epithelial cells were cultured on a collagen gel (the amount of collagen contained in the collagen gel: 4 mg/ml) for 5 days, and then CLiP was inoculated. Two days later, human recombinant oncostatin M (hOSM) (manufactured by R&D Systems Inc.) was added, and then 1 day later, a collagen gel containing 40% EHSgel (Matrigel) was overlaid to perform coculture. The human reprogramming hepatocytes (human chemically induced hepatocyte progenitors: hCLiP) are human liver progenitor cells established from human frozen hepatocytes (Katsuda T. et al. Cell Stem Cell 2017; eLife 2019).

(A) It can be seen that the contact surface between murine BEC (the range indicated in red) and human CLiP has been formed during the early stage of coculture (see FIG. 10A).
(B) One week after overlaying a collagen gel containing EHSgel (Matrigel), CLF was added. In the vicinity of the contact surface between murine BEC and CLiP, CLF flowing into the bile duct can be observed, and it was found that CLF taken up by CLiP has been transported to a murine bile duct tissue (see FIG. 10B).

### Test Example 12: Investigation of construction of an in vitro fatty liver model

Using the liver tissue according to the present invention, as one of attempts to produce injury models, effects of addition of fatty acids to the medium on hepatocytes were investigated. By adding fatty acids to the culture, injury to hepatocytes and accumulation of fat were investigated. As the fatty acids, oleic acid and palmitic acid, which are accumulated in hepatocytes in the case of steatohepatitis, were used. Specifically, the bile duct epithelial cells (BEC) used in the above-mentioned "2. Culture" were cultured on a collagen gel, and to this culture solution, the small hepatocytes (SH) used in the above-mentioned "2. Culture" were added, followed by overlaying 1.6 mg/ml of a type I collagen gel containing 20% (v/v) Matrigel (registered trademark) (manufactured by Corning Incorporated) (EHS-gel). Furthermore, after culture at 37°C for 10 days, 0.5 mM oleic acid (OA) and 0.5 mM palmitic acid (PA) were added to perform culture.

The culture supernatant was recovered over time, and the activity of GOT being an injury marker of hepatocytes was measured. With addition of fatty acids (oleic acid and palmitic acid), the GOT activity can be observed in the culture supernatant compared with no addition (control), and thus it can be seen that hepatocytes have been continuously injured (see FIG. 11).

After culture for 3 days by adding fatty acids to the medium, accumulation of fat droplets was investigated by using Nile Red. The one with addition of fatty acids showed that fat droplets stained with Nile Red have been accumulated (the red region was increased) in the cytoplasm of hepatocytes, compared with the one without addition of fatty acids (see FIG. 12), and it is considered that it is possible to construct an in vitro fatty liver model using the liver tissue of the present invention.

## Claims

1. A method for forming a hepatic epithelioid tissue having a structure of connections between hepatocytes and bile duct epithelial cells, comprising the following steps of:
a step of culturing bile duct epithelial cells on a collagen gel; and
a step of inoculating and culturing hepatocytes on the cultured bile duct epithelial cells.

2. The method for forming a hepatic epithelioid tissue according to claim 1, wherein the collagen gel comprises 2 to 6 mg/mL of collagen.

3. The method for forming a hepatic epithelioid tissue according to claim 1 or 2, wherein the hepatocytes are inoculated and cultured on the bile duct epithelial cells having a density of 20 to 40% of the cultured region as a result of culture on the collagen gel.

4. The method for forming a hepatic epithelioid tissue according to any one of claims 1 to 3, further comprising a step of culturing by overlaying a gel comprising an EHS-gel after the culture step of inoculating and culturing hepatocytes.

5. The method for forming a hepatic epithelioid tissue according to claim 4, wherein the gel comprises the EHS-gel at a concentration of 5% (v/v) or more.

6. The method for forming a hepatic epithelioid tissue according to any one of claims 1 to 5, wherein the hepatocytes are small hepatocytes and/or mature hepatocytes.

7. The method for forming a hepatic epithelioid tissue according to any one of claims 1 to 5, wherein the hepatocytes are murine small hepatocytes and/or murine mature hepatocytes.

8. The method for forming a hepatic epithelioid tissue according to any one of claims 1 to 5, wherein the hepatocytes are human liver progenitor cells.

9. A hepatic epithelioid tissue formed by the method for forming a hepatic epithelioid tissue according to any one of claims 1 to 8.

10. A liver tissue comprising the hepatic epithelioid tissue according to claim 9.

11. A liver tissue reconstructed ex vivo having a structure of connections between hepatocytes and bile duct epithelial cells.

12. The liver tissue according to claim 10 or 11, wherein one or more markers selected from the group consisting of the following (a) to (d) are expressed:
(a) one or more hepatocyte metabolic function markers selected from the group consisting of Cps, Tdo2, Cyp, and Ugt1a1;
(b) one or more bile duct epithelial cell transporter markers selected from the group consisting of Ae2 and Cftr;
(c) one or more bile duct epithelial cell cytoskeleton markers selected from the group consisting of Ck7 and Ck19; and
(d) Cldn4 being a molecule that forms a tight junction of a bile duct.

13. The liver tissue according to any one of claims 10 to 12, wherein one or more hepatocyte metabolic function markers selected from the group consisting of Cps, Tdo2, Cyp, and Ugt1a1 are expressed.

14. The liver tissue according to any one of claims 10 to 13, wherein one or more bile duct epithelial cell transporter markers selected from the group consisting of Ae2 and Cftr are expressed.

15. The liver tissue according to any one of claims 10 to 14, wherein one or more bile duct epithelial cell cytoskeleton markers selected from the group consisting of Ck7 and Ck19 are expressed.

16. The liver tissue according to any one of claims 10 to 15, wherein Cldn4 being a molecule that forms a tight junction is expressed.

17. The liver tissue according to any one of claims 10 to 16, which is used as a disease model.

18. The liver tissue according to claim 17, wherein the disease model is a fatty liver model.
